# EUROPÄISCHE PATENTSCHRIFT

(11) **EP 3 243 542 B1**
(45) Veröffentlichungstag und Bekanntmachung des Hinweises auf die Patenterteilung: **05.12.2018**
(21) Anmeldenummer: 16178695.9
(22) Anmeldetag: 08.07.2016
(51) Int. Cl.: A61M 5/32

(54) **STECHMITTELSCHUTZVORRICHTUNG FÜR EINE SPRITZE**
PIERCING TOOL PROTECTION DEVICE FOR A SYRINGE
DISPOSITIF DE PROTECTION DE MOYEN DE PERÇAGE POUR UNE SERINGUE

(30) Priorität: 13.05.2016 DE 102016108870
(43) Veröffentlichungstag der Anmeldung: 15.11.2017
(73) Patentinhaber: Gerresheimer Regensburg GmbH, 93047 Regensburg (DE)
(72) Erfinder: Großer, Jörg, 93138 Lappersdorf (DE)
(74) Vertreter: Hannke, Christian

(56) Entgegenhaltungen:
- US-A1- 2016 106 929

## Beschreibung

Die Erfindung wird durch die Merkmale des Anspruchs 1 definiert und betrifft eine Stechmittelschutzvorrichtung für eine Spritze mit einem Spritzenkörper und einem an dem distalen Ende des Spritzenkörpers angeordneten Stechmittel, umfassend ein sich entlang einer axialen Richtung erstreckendes formstabiles Hülsenelement, welches zumindest teilweise ein sich entlang einer axialen Richtung erstreckendes inneres Element umschließt, wobei das innere Element aus einem elastischen Material besteht und zumindest teilweise das Stechmittel umschließt.

In der Regel finden derartige Stechmittelschutzvorrichtungen Anwendung bei vorgefüllten Spritzen. Die Handhabung derartiger Spritzen ist sehr einfach, da das Medium nicht vor der Anwendung in die Spritze transferiert werden muss. Für Impfstoffe und zahlreiche andere Medikamente sind sie heutzutage das Primärpackmittel erster Wahl. Diese Spritzen sind üblicherweise aus Glas oder Kunststoff (beispielsweise COC, COP) hergestellt und werden mit einem Stechmittel bestückt. In der Regel weisen solche Stechmittel, beispielsweise Kanülen, einen sehr feinen Schliff auf, um eine möglichst schmerzfreie Injektion zu ermöglichen. Solche Schliffe können leicht durch mechanische Einflüsse beschädigt werden, wodurch dem Patienten bei einer Injektion unnötige Schmerzen bereitet werden können. Demnach werden die Stechmittel mit einer Schutzkappe aus einem elastischen Material versehen. Ein solches Flexible Needle Shield (FNS) schützt den feinen Schliff des Stechmittels durch seine elastischen Eigenschaften vor mechanischen Einflüssen. Ferner soll die Sterilität des Stechmittels gewährleistet werden. Das FNS umgreift dazu auch das konische Endstück des Spritzenkörpers. Durch den Sitz des inneren Elements auf dem Endstück des Spritzenkörpers ist das Stechmittel luftdicht abgeschlossen, wodurch die Sterilität des Stechmittels gewährleistet wird.

Ein solches FNS bietet jedoch, bedingt durch seine elastischen Eigenschaften, keinen ausreichenden Schutz vor größeren mechanischen Belastungen. Demzufolge wurden bereits formstabile Hülsenelemente an das FNS angeordnet. Ein solches System bestehend aus einem formstabilen Hülsenelement und einem Flexible Needle Shield (FNS) wird auch als Rigid Needle Shield (RNS) bezeichnet. Problematisch ist hierbei die Verbindung zwischen dem FNS und dem RNS. Bei herkömmlichen Stechmittelschutzvorrichtungen wird das FNS in einer Aufnahme des Hülsenelements gehalten. Dabei rutscht häufig das elastische Element aus der Aufnahme, wodurch dieses von dem formstabilen Hülsenelement getrennt wird. Das formstabile Hülsenelement könnte dabei verloren gehen, wodurch die Gefahr entsteht, dass das Stechmittel beschädigt wird. Zudem kann das FNS ohne umgebende stabile Komponente durchstochen werden und stellt somit ein Risiko hinsichtlich Nadelstichverletzungen für den Benutzer dar.

In der US 2016//0106929 A1 wird beispielsweise ein solches formstabiles Hülsenelement offenbart. Ferner wird eine FNS gezeigt, welches einen radial umlaufenden Vorsprung aufweist. Das FNS wird in dem Hülsenelement durch Greifarme gehalten, welche den Vorsprung des FNS umgreifen. Die Gefahr eines Abrutschen des FNS besteht auch bei dieser Anordnung.

Aufgabe der vorliegenden Erfindung ist es demnach, eine Stechmittelschutzvorrichtung bereitzustellen, durch welche die eingangs genannten Nachteile umgangen werden und welche konstruktiv einfach und kostengünstig herzustellen ist. Ferner ist es Aufgabe der Erfindung, eine Spritze bereitzustellen, welche mit einer solchen Stechmittelschutzvorrichtung ausgestattet ist.

Diese Aufgaben wird gelöst von einer Stechmittelschutzvorrichtung für eine Spritze mit einem Spritzenkörper und einem an dem distalen Ende des Spritzenkörpers angeordneten Stechmittel, umfassend ein sich entlang einer axialen Richtung (X) erstreckendes formstabiles Hülsenelement, welches zumindest teilweise ein sich entlang einer axialen Richtung (X) erstreckendes inneres Element umschließt, wobei das innere Element aus einem elastischen Material besteht und zumindest teilweise das Stechmittel umschließt. Die Stechmittelschutzvorrichtung zeichnet sich dadurch aus, dass ein mit dem Hülsenelement verbundenes Verbindungselement zumindest einen sich in axialer Richtung (X) erstreckenden Vorsprung aufweist, welcher zumindest abschnittsweise in einer Ausnehmung des inneren Elements aufgenommen ist, so dass eine kraft- und / oder formschlüssige Verbindung zwischen dem Hülsenelement und dem inneren Element besteht.

Das formstabile Hülsenelement stellt einen ausreichenden Schutz für das Stechmittel hinsichtlich mechanischer Belastungen dar. Ferner wird das Abziehen der Stechmittelschutzvorrichtung erleichtert, da der Nutzer die Stechmittelschutzvorrichtung besser greifen kann. Der Vorsprung des Verbindungselements wird demnach in der Aufnahme des inneren Elements aufgenommen wodurch dieses druckbeaufschlagt wird, somit wird durch das Verbindungselement sichergestellt, dass eine mechanisch feste Verbindung zwischen dem inneren Element und dem Hülsenelement besteht. Ein unerwünschtes Herausrutschen des inneren Elements aus dem Hülsenelement wird somit effektiv unterdrückt.

Durch das innere Element wird das Stechmittel insbesondere der Schliff des Stechmittels vor mechanischen Einflüssen bzw. Beschädigungen geschützt. Das Stechmittel kann beispielsweise eine Kanüle, eine Nadel oder auch eine Lanzette sein.

Vorzugsweise ist der Spritzenkörper als hohler Kreiszylinder ausgestaltet und weist in seinem distalen Endbereich ein konisches Endstück auf, an welchem das Stechmittel angeordnet ist.

Das distale Ende einer Spritze ist dem Applikationsort am nächsten. Somit ist am distalen Ende das Stechmittel angeordnet. Am proximalen Ende sind in der Regel die Betätigungselemente, beispielsweise der Plunger angeordnet. Für die Stechmittelschutzvorrichtung sind die Begriffe "distal" und "proximal" analog zu verstehen. Die axiale Richtung (X) ist weiterhin in eine distale Richtung (X₁) und eine proximale Richtung (X₂) unterteilt. Eine radiale Richtung (R) verläuft senkrecht zu der axialen Richtung (X).

Vorzugsweise besteht der Spritzenkörper aus Glas oder aus einem Polymer-Kunststoff, bevorzugt einem Polyolefin, beispielsweise Polypropylen oder Polyethylen, besonders bevorzugt aus einem Cyclo-Olefinen Polymer (COP) beziehungsweise aus einem Cyclo-Olefinen Co-Polymer (COC).

Bevorzugt ist das innere Element im Wesentlichen zylindrisch ausgestaltet ist. Besonders bevorzugt weist das innere Element dabei eine im Wesentlichen kreisförmige Grundfläche auf. Vorteilhafterweise weist das innere Element an seinem proximalen Ende eine weitere Ausnehmung auf, an welcher das Stechmittel der Spritze anordenbar ist. Bevorzugt weist die weitere Ausnehmung einen ersten Abschnitt auf, welcher einen konstanten Innendurchmesser aufweist und in dem ein distales Endstück des Spritzenkörpers aufnehmbar ist. Ferner ist es bevorzugt, dass die weitere Ausnehmung einen zweiten Abschnitt aufweist, welcher einen sich in axialer Richtung (X) verkleinernden Innendurchmesser aufweist.

Vorteilhafterweise liegt das innere Element dichtend an dem distalen Endbereich des Spritzenkörpers an beziehungsweise ist das innere Element teilweise über dem distalen Endbereich beziehungsweise dem konischen Endstück des Spritzenkörpers angeordnet. Das Stechmittel ist somit luftdicht eingeschlossen und vor Kontamination geschützt. Die Sterilität des Stechmittels ist demnach gewährleistet.

Nach einer besonders bevorzugten Ausführungsform ist das Hülsenelement im Wesentlichen als kreisförmiger Hohlzylinder ausgebildet und weist ein distales sowie ein proximales Ende auf. Bevorzugt weist der Hohlzylinder eine geschlossene Wandung auf. Es wäre jedoch auch denkbar, dass die Wandung Schlitze oder Ausnehmungen aufweist. Das Hülsenelement könnte also auch eine flügelartige Wandung aufweisen. Vorzugsweise ist das Verbindungselement an dem distalen Ende des Hülsenelements angeordnet. Bevorzugt ist die Verbindung zwischen dem Hülsenelement und dem Verbindungselement eine kraft- und / oderform- und / oder stoffschlüssige Verbindung. Eine solche formschlüssige Verbindung könnte beispielsweise eine Clipsverbindung oder eine Nut-Feder-Verbindung sein. Eine mögliche stoffschlüssige Verbindung könnte beispielsweise eine Klebe- oder Schweißverbindung sein. Eine mögliche kraftschlüssige Verbindung könnte beispielsweise ein Schraubverbindung oder eine Reibverbindung sein.

Vorzugsweise weist das innere Element an seinem distalen Ende ein sich in radialer Richtung (R) erstreckendes Flanschelement auf, welches in einer Aufnahmeeinrichtung des Hülsenelements aufgenommen ist. Die Aufnahmeeinrichtung ist vorteilhafterweise an dem distalen Ende des Hülsenelements angeordnet. Vorzugsweise sind die Aufnahmeeinrichtung und das Hülsenelement einstückig ausgebildet. Es wäre jedoch auch denkbar, dass die Aufnahmeeinrichtung ein separates Bauteil ist und mit dem Hülsenelement verbunden ist. Eine solche Verbindung könnte eine Clipsverbindung, eine Klebeverbindung oder eine Schweißverbindung sein.

Ferner weist die Aufnahmeeinrichtung vorteilhaft eine erste Wandung auf, welche sich ausgehend von dem distalen Ende des Hülsenelements in axialer Richtung (X) erstreckt, sowie eine zweite Wandung, welche sich im Wesentlichen in radialer Richtung erstreckt. Bevorzugt liegt das Flanschelement auf der zweiten Wandung der Aufnahmeeinrichtung auf, wodurch das innere Element, insbesondere beim Abziehen der Stechmittelschutzvorrichtung in axialer Richtung beziehungsweise in distaler Richtung gehalten wird. Vorzugsweise umläuft das Flanschelement das innere Element vollständig. Es wäre jedoch auch denkbar, dass das Flanschelement nur abschnittsweise an der äußeren Oberfläche des inneren Elements angeordnet ist. Ferner ist es bevorzugt, dass die Aufnahmeeinrichtung zylinderartig ausgebildet ist und somit eine geschlossene erste Wandung aufweist. Es wäre jedoch auch denkbar, dass die erste Wandung Ausnehmungen oder Schlitze aufweist.

Gemäß einer weiteren bevorzugten Ausführungsform weist das Verbindungselement ein kreisförmiges Basiselement auf, an welchem der Vorsprung (9) angeordnet ist. Bevorzugt weist das kreisförmige Basiselement eine radiale Außenfläche auf, welche mit dem Hülsenelement verbunden ist. Durch die vorteilhafte Anordnung des Verbindungselements am distalen Ende des Hülsenelements schließt das Verbindungselement das Hülsenelement am distalen Ende ab. Das Verbindungselement ist sozusagen auch eine Art Deckelelement. Es wird somit einerseits das innere Element in axialer beziehungsweise distaler Richtung fixiert und andererseits wird ein Schutz gegen mechanische Einflüsse in dieser Richtung bereitgestellt.

Nach einer besonders bevorzugten Ausführungsform ist der Vorsprung an dem Verbindungselement mittig bezüglich einer Mittelachse (M_{H}) des Hülsenelements angeordnet. Vorzugsweise ist die Ausnehmung mittig bezüglich einer Mittelachse (M_{IE}) des inneren Elements angeordnet. Durch die jeweilige mittige Anordnung und die Verbindung zwischen dem Verbindungselement und dem inneren Element ist vorteilhaft das innere Element bezüglich des Hülsenelements zentrierbar. Das Stechmittel ist somit zentrisch in dem inneren Element angeordnet und somit keinen mechanischen Belastungen in radialer Richtung ausgesetzt.

Vorteilhafterweise ist der Vorsprung des Verbindungselements konisch ausgebildet. Bevorzugt verjüngt sich dabei der Vorsprung ausgehend von dem kreisförmigen Basiselement in axialer Richtung (X). Besonders bevorzugt ist der Vorsprung als konischer Hohlzylinder ausgebildet. Vorzugsweise ist ein Außendurchmesser eines ersten Bereichs des Vorsprungs größer als ein Innendurchmesser der Ausnehmung des inneren Elements. Vorzugsweise ist durch die Aufnahme des Vorsprungs in der Ausnehmung des inneren Elements das innere Element elastisch verformt. Durch diese Verformung ist vorteilhaft das Flanschelement des inneren Elements in radialer Richtung (R) an die Aufnahmeeinrichtung gedrückt. Bevorzugt wird durch diese Verformung das Flanschelement des inneren Elements in radialer Richtung (R) an die erste Wandung der Aufnahmeeinrichtung gepresst. Durch diese Ausführungsform wird auf einfache Weise ein Herausrutschen des inneren Elements in proximaler Richtung verhindert. Zum einen besteht eine kraft- und / oder formschlüssige Verbindung zwischen dem Vorsprung des Verbindungselements und der Ausnehmung des inneren Elements. Darüber hinaus wird das Flanschelement gegen die erste Wandung der Aufnahmeeinrichtung gepresst, wodurch ein Kraftschluss zwischen dem Flanschelement und der ersten Wandung des inneren Elements besteht. Ferner wird eine Stirnfläche der zweiten Wandung gegen einen Abschnitt des inneren Elements gedrückt, welcher in proximaler Richtung unterhalb des Flanschelements liegt. Somit wird ein Abrutschen des Flanschelements über die zweite Wandung effektiv verhindert.

Nach eine bevorzugten Ausführungsform ist an dem Basiselement des Verbindungselements zumindest ein axiales Toleranzausgleichselement angeordnet, welches sich in axialer Richtung (X) zu dem inneren Element hin erstreckt und das innere Element kontaktiert. Das zumindest eine axiale Toleranzausgleichselement kann das elastische innere Element demnach punktuell verformen/ komprimieren oder dieses sogar penetrieren. Somit werden bauliche Toleranzen des inneren Elements, des Hülsenelements und des Endstücks des Spritzenkörpers effektiv ausgeglichen. Ferner stellt das Toleranzausgleichselement zusätzlich ein Fixierung in axialer und in radialer Richtung für das innere Element dar.

Vorzugsweise sind zumindest zwei axiale Toleranzausgleichselemente an dem Basiselement des Verbindungselements angeordnet. Besonders bevorzugt sind sechs axiale Toleranzausgleichselemente an dem Basiselement des Verbindungselements angeordnet. Vorteilhafterweise sind die zumindest zwei beziehungsweise sechs axialen Toleranzausgleichselemente kreisförmig um den Vorsprung angeordnet. Diese Toleranzausgleichselemente sind vorteilhaft als kegelförmige Vorsprünge beziehungsweise Dorne ausgestaltet.

Nach einer weiteren vorteilhaften Ausführungsform ist das axiale Toleranzausgleichselement als ein den Vorsprung umlaufendes ringartiges Element ausgestaltet. Vorzugsweise verjüngt sich das ringartige Element in axialer Richtung (X) zu dem inneren Element hin. Das ringartige Element weist somit eine breite Grundfläche auf, welche an dem kreisförmigen Basiselement angeordnet ist und eine schmale Kante, welche mittig über der Grundfläche liegt und das innere Element verformt/ komprimiert oder penetriert.

Nach einer weiteren Ausführungsform umfasst der Vorsprung eine Außenfläche, an welcher in axialer Richtung verlaufende Rippen angeordnet sind. Diese Rippen können das elastische Material des inneren Elements verformen / komprimieren oder penetrieren. Zwischen dem inneren Element und dem Verbindungselement kann somit eine stabilere Verbindung realisiert werden.

Vorzugsweise besteht das elastische innere Element aus Gummi oder einem elastischen synthetischen Elastomer. Bevorzugt bestehen das Hülsenelement und das Verbindungselement aus einem thermoplastischen Kunststoff. Vorzugsweise sind diese Elemente mittels eines Ein- oder Mehrkomponenten-Spritzgussverfahrens hergestellt. Die Elemente können damit kostengünstig mit nur einem Werkzeug in einem Arbeitsgang hergestellt werden

Nach einer weiteren vorteilhaften Ausführungsform ist das Hülsenelement mit einer Oberfläche ausgestattet, welche rutschfest ist und / oder die Haptik verbessert. Dies kann beispielsweise durch eine Beschichtung erfolgen oder durch eine Gummierung. Es könnten auch Griffelemente, wie beispielsweise Vorsprünge oder Ausnehmungen vorgesehen sein.

Die Aufgabe wird auch durch eine Spritze insbesondere eine vorfüllbare Spritze ausgestattet mit einer Stechmittelschutzvorrichtung nach einem der vorhergehenden Ausführungsformen gelöst.

Vorzugsweise ist der Spritzenkörper als hohler Kreiszylinder ausgestaltet, in dem ein Kolben beweglich angeordnet ist. Bevorzugt weist die Spritze in seinem distalen Endbereich ein konisches Endstück auf, an welchem das Stechmittel angeordnet ist. Vorzugsweise besteht der Spritzenkörper aus Glas oder aus einem Polymer-Kunststoff, bevorzugt einem Polyolefin, beispielsweise Polypropylen oder Polyethylen, besonders bevorzugt aus einem Cyclo-Olefinen Polymer (COP) beziehungsweise aus einem Cyclo-Olefinen Co-Polymer (COC).

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig. 1: eine Schnittdarstellung einer Stechmittelschutzvorrichtung;
- Fig. 2: eine perspektivische Ansicht eines Verbindungselements;
- Fig. 3: eine Schnittdarstellung des Verbindungselements aus Fig. 2;
- Fig. 4: eine perspektivische Ansicht eines weiteren Verbindungselements;
- Fig. 5: eine perspektivische Ansicht des inneren Elements;
- Fig. 6: eine Schnittdarstellung des inneren Elements;
- Fig. 7: eine Schnittdarstellung des Hülsenelements;
- Fig. 8: eine perspektivische Ansicht des Hülsenelements;
- Fig. 9: eine weitere perspektivische Ansicht des Hülsenelements;
- Fig. 10: eine Seitenansicht einer Spritze.

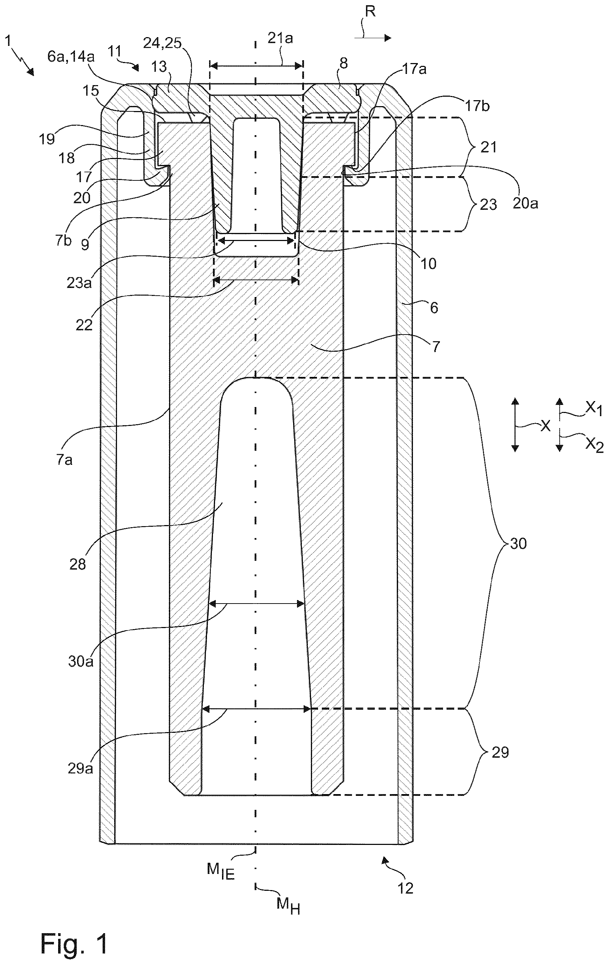

Fig. 1 zeigt eine Stechmittelschutzvorrichtung (1) für eine Spritze (2) mit einem Spritzenkörper (3) und einem an dem distalen Ende (4) des Spritzenkörpers (3) angeordneten Stechmittel (5), umfassend ein sich entlang einer axialen Richtung (X) erstreckendes formstabiles Hülsenelement (6), welches zumindest teilweise ein sich entlang einer axialen Richtung (X) erstreckendes inneres Element (7) umschließt. wobei das innere Element (7) aus einem elastischen Material besteht und zumindest teilweise das Stechmittel (5) umschließt, wobei ein mit dem Hülsenelement (6) verbundenes Verbindungselement (8) zumindest einen sich in axialer Richtung (X) erstreckenden Vorsprung (9) aufweist, welcher zumindest abschnittsweise in einer Ausnehmung (10) des inneren Elements (7) aufgenommen ist, so dass eine kraft- und / oder formschlüssige Verbindung zwischen dem Hülsenelement (6) und dem inneren Element (7) besteht.

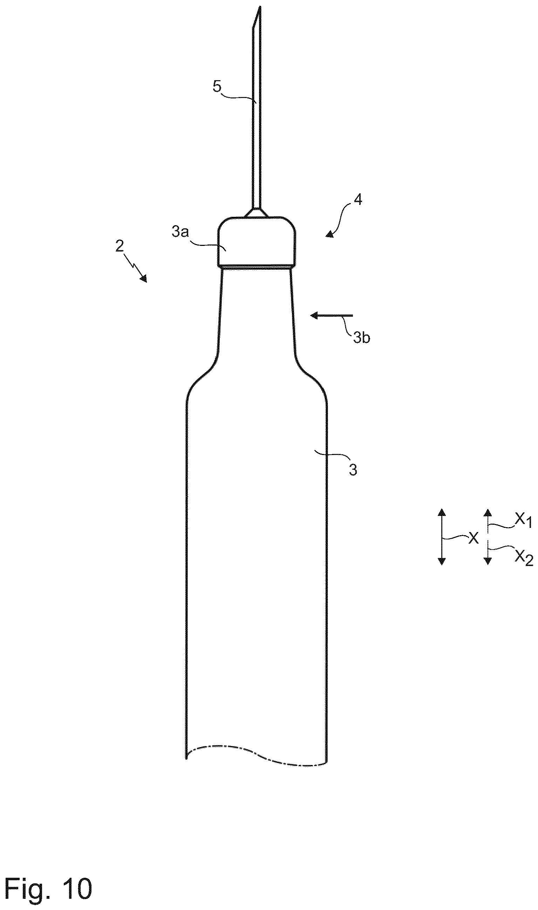

In Fig. 10 ist eine typische Spritze gezeigt, welche mit einer Stechmittelschutzvorrichtung versehen werden kann. Die Spritze (2) umfasst einen Spritzenkörper (3), welcher als hohler Kreiszylinder ausgestaltet ist. Der Spritzenkörper weist einen distalen Endbereich (3b) mit einem distalen Ende (4) auf. An dem distalen Ende (4) ist ein Stechmittel (5) angeordnet. Dieses Stechmittel (5) ist über eine Bohrung in dem distalen Endbereich (3b) mit dem Hohlraum des Spritzenkörpers (3) verbunden, so dass das zu injizierende Medium bei einer Anwendung der Spritze (2) aus dem Hohlraum durch das Stechmittel (5) treten kann. Der distale Endbereich (3b) ist als konisches Endstück ausgestaltet, welches einen kleineren Außendurchmesser als der Spritzenkörper (3) aufweist. Weiterhin weist die Spritze einen Übergangsbereich auf, in dem der Außendurchmesser des Spritzenkörpers (3) in den Außendurchmesser des Endstücks übergeht.

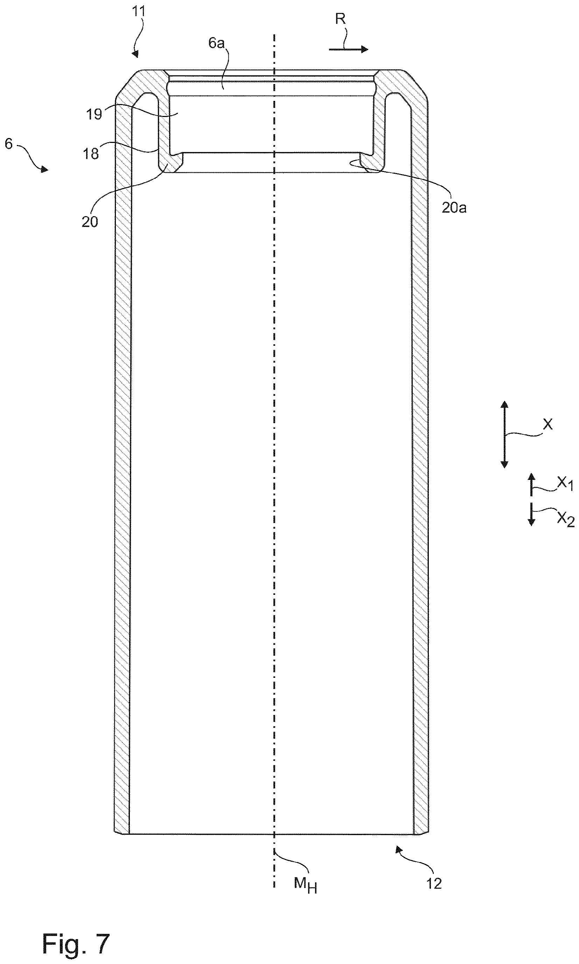

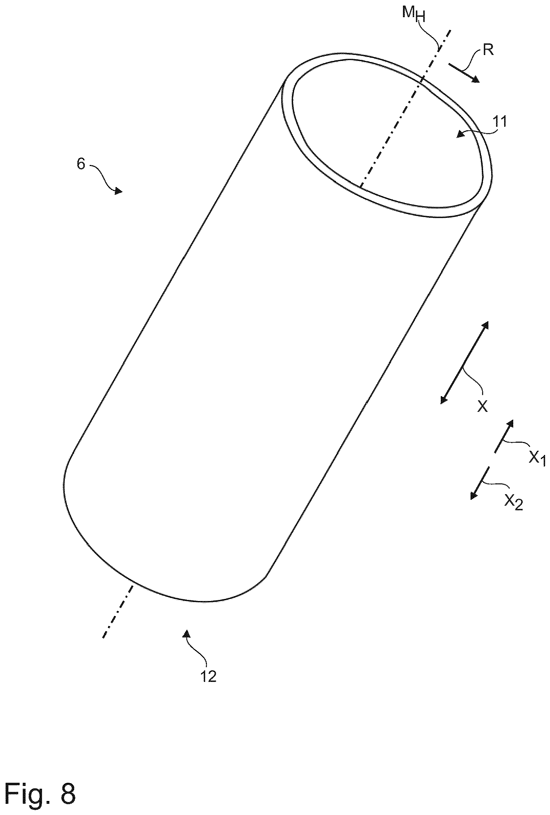

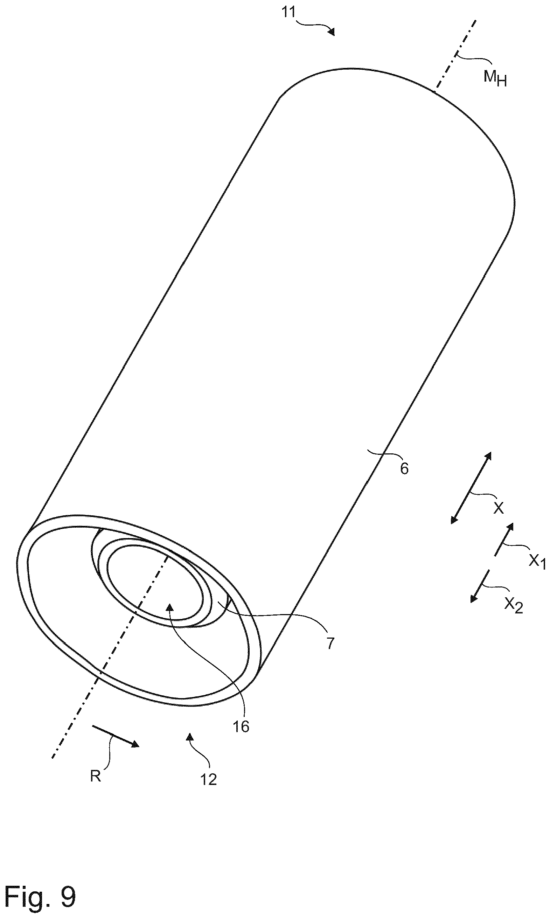

Das Hülsenelement (6) ist als kreisförmiger Hohlzylinder ausgebildet und weist ein distales (11) sowie ein proximales (12) Ende auf. Dies ist in den Figuren 1, 7, 8 und 9 zu erkennen. Fig. 1 zeigt eine Schnittdarstellung der Stechmittelschutzvorrichtung (1) mit dem Hülsenelement (6), dem inneren Element (7) und dem Verbindungselement (8). In Fig. 7 ist lediglich das Hülsenelement (6), in einer Schnittdarstellung gezeigt. In Fig. 8 ist das Hülsenelement (6) in einer perspektivischen Ansicht dargestellt, wobei insbesondere das distale Ende (11) des Hülsenelements (6) erkennbar ist. In Fig. 9 ist das Hülsenelement (6) in einer weiteren perspektivischen Ansicht dargestellt, wobei insbesondere das proximale Ende (12) des Hülsenelements (6) erkennbar ist. Ferner ist das proximale Ende (16) des inneren Elements (7) zu erkennen.

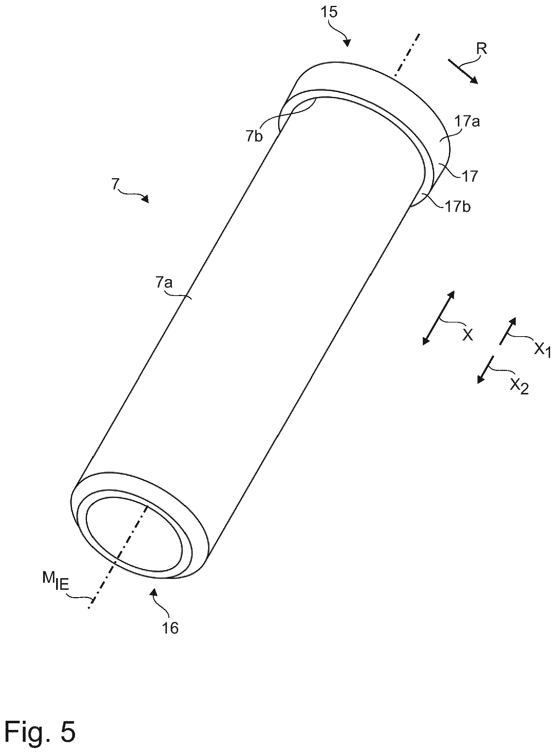

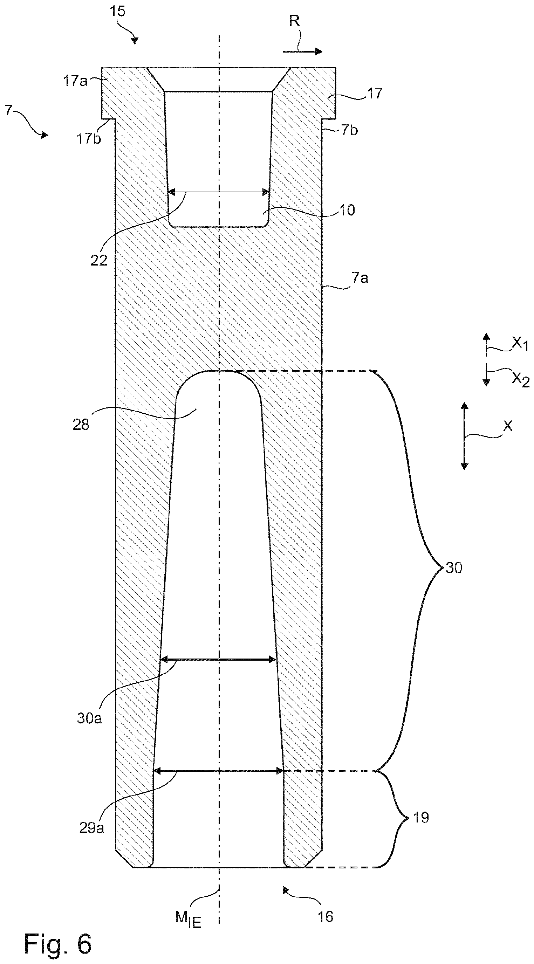

Das innere Element (7) ist in den Figuren 1 und 6 in einer Schnittdarstellung gezeigt. In Fig. 5 wird das innere Element (7) perspektivisch dargestellt. Das innere Element (7) ist als kreisförmiger Zylinder ausgestaltet und weist an seinem distalen Ende (15) eine zylindrische Ausnehmung beziehungsweise ein Sackloch (10) auf. An seinem proximalen Ende (16) weist das innere Element (7) weiterhin eine weitere Ausnehmung (28) auf, in welcher das Stechmittel (5) der Spritze (3) anordenbar ist.

Die weitere Ausnehmung (28) weist einen ersten Abschnitt (29) mit einen konstanten Innendurchmesser (29a) auf, in dem ein distales Endstück (3a) des Spritzenkörpers (3) aufnehmbar ist. Ferner weist die weitere Ausnehmung (28) einen zweiten Abschnitt (30) mit einem einen sich in axialer Richtung (X) verkleinernden Innendurchmesser (30a) auf. Durch die Aufnahme des distalen Endstücks (3a) des Spritzenkörpers (3) in dem inneren Element (7) kann das Stechmittel (5) steril abgeschlossen werden.

Ferner weist das innere Element (7) an seinem distalen Ende (15) ein sich in radialer Richtung (R) erstreckendes Flanschelement (17) auf. Das Flanschelement (17) ist umlaufend an der Außenfläche (7a) des inneren Elements (7) angeordnet und umfasst eine sich in axialer Richtung (X) erstreckende Außenfläche (17a) und eine sich in radialer Richtung (R) erstreckende Auflagefläche (17b).

Das innere Element (7) beziehungsweise dessen Flanschelement (17) ist in einer Aufnahmeeinrichtung (18) des Hülsenelements (6) aufgenommen. Die Aufnahmeeinrichtung (18) ist zylindrisch und einstückig mit dem Hülsenelement (6) ausgebildet. Die Aufnahmeeinrichtung (18) ist an dem distalen Ende (11) des Hülsenelements (8) angeordnet und umfasst sowohl eine erste Wandung (19), welche sich ausgehend von dem distalen Ende (11) des Hülsenelements (8) in axialer Richtung (X) erstreckt als auch eine zweite Wandung (20), welche sich im Wesentlichen in radialer Richtung (R) nach innen zu einer Mittelachse (M_{IE}) des inneren Elements (7) erstreckt. Die Auflagefläche (17b) des inneren Elements (7) liegt dabei auf der zweiten Wandung (20) auf. Ferner erstreckt sich die zweite Wandung (20 ausgehend von der ersten Wandung (18) nach innen hin zur Mittelachse (M_{IE}) mit einer Steigung in distaler Richtung (X₁).

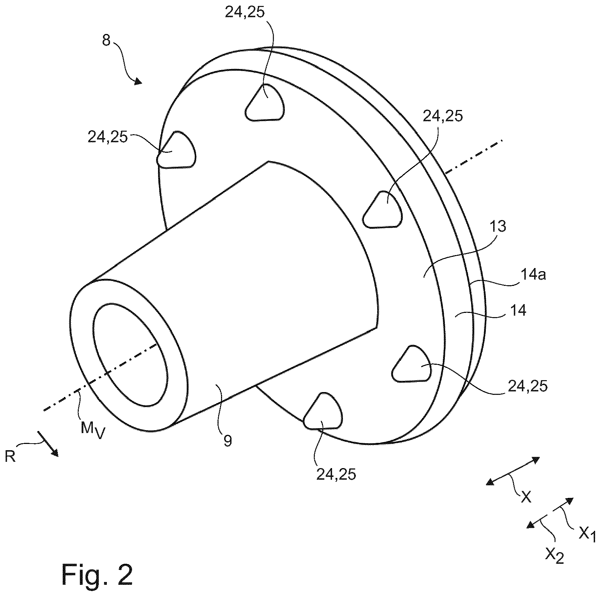

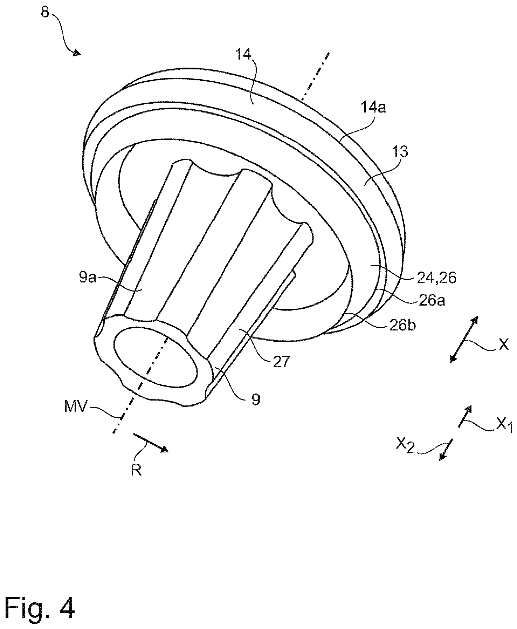

In Fig. 1 ist weiterhin das Verbindungselement (8) dargestellt, welches an dem distalen Ende (11) des Hülsenelements (6) angeordnet beziehungsweise mit diesem verbunden ist. Diese Verbindung kann eine kraft- und / oder form- und / oder stoffschlüssige Verbindung sein. Ferner ist das Verbindungselement (8) kraft- und / oder formschlüssig mit dem inneren Element (7) verbunden. Die Figuren 2 und 4 zeigen ein Verbindungselement (8) gemäß einer ersten Ausführungsform und Fig. 4 zeigt ein Verbindungselement (8) gemäß einer weiteren Ausführungsform.

Das Verbindungselement (8) umfasst ein kreisförmiges Basiselement (13) mit einer Mittelachse (M_{V}). An dem Basiselement (13) ist der Vorsprung (9) mittig bezüglich der Mittelachse (M_{V}) angeordnet. Das kreisförmige Basiselement (13) weist weiterhin eine radiale Außenfläche (14) auf, welche mit dem Hülsenelement (6) verbunden ist. Insbesondere ist an der radialen Außenfläche (14) ein Vorsprung (14a) ausgebildet, welcher in einer Nut (6a) des Hülsenelements aufgenommen ist. Dadurch wird eine Clipsverbindung zwischen dem Hülsenelement (6) und dem Verbindungselement (8) realisiert.

Der Vorsprung (9) des Verbindungselements (8) ist konisch ausgebildet. Demnach ist ein Außendurchmesser (21a) eines ersten Bereichs (21) des Vorsprungs (9) größer als ein Innendurchmesser (22) der Ausnehmung (10) des inneren Elements (7). Ferner ist ein Außendurchmesser (23a) eines zweiten Bereichs (23) des Vorsprungs (9) kleiner als ein Innendurchmesser (22) der Ausnehmung (10). Der Vorsprung ist weiterhin als konischer Hohlzylinder ausgebildet, der an seinem distalen Ende durch das Basiselement (13) abgeschlossen ist.

Durch die Aufnahme des Vorsprungs (9) in der Ausnehmung (10) des inneren Elements (7) ist das innere Element (7) elastisch verformt. Durch eine solche Übermaßpassung wirkt eine elastische Kraft zwischen dem inneren Element (7) und dem Verbindungselement (8) beziehungsweise dem Hülsenelement (6). Ferner wird durch diese Verformung das Flanschelement (17) des inneren Elements (7) in radialer Richtung (R) an die Aufnahmeeinrichtung (18) gedrückt. Insbesondere wird die Außenfläche (7a) des inneren Elements (7) gegen die erste Wandung (19) der Aufnahmeeinrichtung (18) gepresst. Somit ist eine weitere kraftschlüssige Verbindung zwischen dem inneren Element (7) und dem Hülsenelement (6) gegeben.

Ferner wird eine Stirnfläche (20a) der zweiten Wandung (20) gegen einen Abschnitt (7b) des inneren Elements (7) gedrückt, welcher in proximaler Richtung (X₂) unterhalb des Flanschelements (17) liegt. Zum einen wird hierdurch eine weitere kraftschlüssige Verbindung zwischen dem inneren Element (7) und dem Hülsenelement beziehungsweise der zweiten Wandung (20) erreicht. Zum anderen wird durch die konische Form des Vorsprungs (9) und die Steigung der zweiten Wandung (20) das Flanschelement derart verformt, dass es sich der zweiten Wandung annähert beziehungsweise anliegt. Die Auflagefläche (17b) des Flanschelements (17) verläuft also nach der Verformung in radialer Richtung nach außen mit einer Steigung in proximaler Richtung. Mit anderen Worten verhaken sich die Aufnahmeeinrichtung (18) und das innere Element in axialer Richtung (X). Demnach ist eine besonders feste Verbindung zwischen dem inneren Element (7) und dem Hülsenelement geschaffen.

Der Vorsprung (9) an dem Verbindungselement (8) ist mittig bezüglich einer Mittelachse (M_{H}) des Hülsenelements (6) angeordnet. Die Ausnehmung (10) ist mittig bezüglich einer Mittelachse (M_{IE}) des inneren Elements (7) angeordnet. Durch die jeweilige mittige Anordnung und die Verbindung zwischen dem Verbindungselement (8) und dem inneren Element (7) ist das innere Element (7) bezüglich des Hülsenelements (6) zentrierbar.

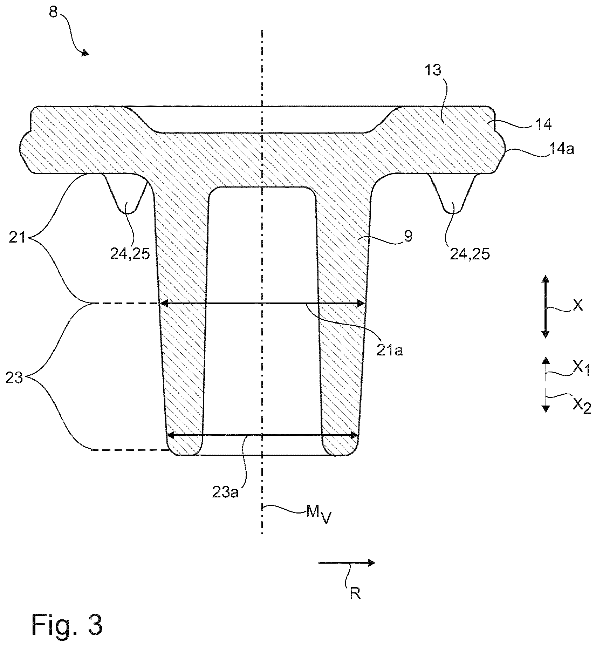

Das Verbindungselement (8) weist weiterhin an dem Basiselement (13) des Verbindungselements (8) zumindest ein axiales Toleranzausgleichselement (24) auf. Gemäß der in den Figuren 2 und 3 gezeigten Ausführungsform sind an dem kreisförmigen Basiselement (13) sechs axiale Toleranzausgleichselemente (24) angeordnet. Diese Toleranzausgleichselemente (24) sind kreisförmig um den Vorsprung (9) beziehungsweise die Mittelachse (M_{V}) angeordnet. Ferner sind diese Toleranzausgleichselemente (24) als kegelförmige Vorsprünge (25) beziehungsweise Dorne ausgestaltet, welche sich in proximaler Richtung (X₂) zu dem inneren Element (7) hin erstrecken und das innere Element (7) kontaktieren beziehungsweise verformen oder gar penetrieren.

Gemäß einer weiteren Ausführungsform ist das axiale Toleranzausgleichselement (24) als ein den Vorsprung (9) umlaufendes ringartiges Element (26) ausgestaltet, wobei das ringartige Element (26) sich in axialer Richtung (X) zu dem inneren Element (7) beziehungsweise in proximaler Richtung (X₂) hin verjüngt. Das ringartige Element (26) weist somit eine breite Grundfläche (26a) auf, welche an dem kreisförmigen Basiselement (13) angeordnet ist. Drüber hinaus weist das ringartige Element (26) eine schmale Kante (26b) auf, welche mittig über der Grundfläche (26a) liegt und das innere Element (7) verformt/ komprimiert oder penetriert.

Bei der Ausführungsform gemäß Fig. 4 umfasst der Vorsprung (9) eine Außenfläche (9a), an welcher Rippen angeordnet sind. Die Rippen (27) verlaufen in axialer Richtung (X) und erstrecken sich in radialer Richtung (R) nach außen. Diese Rippen (27) können das elastische Material des inneren Elements (7) verformen / komprimieren oder penetrieren. Zwischen dem inneren Element (7) und dem Verbindungselement (8) kann somit eine stabilere Verbindung realisiert werden.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Vorrichtung
- 2: Spritze
- 3: Spritzenkörper
- 3a: distales Endstück des Spritzenkörpers
- 3b: distalen Endbereich des Spritzenkörpers
- 4: distales Ende des Spritzenkörpers
- 5: Stechmittel
- 6: Hülsenelement
- 6a: Nut in Hülsenelement
- 7: inneres Element
- 7a: Außenfläche des inneren Elements
- 7b: Abschnitt des inneren Elements
- 8: Verbindungselement
- 9: Vorsprung am Verbindungselement
- 9a: Außenfläche des Vorsprungs
- 10: Ausnehmung des inneren Elements
- 11: distales Ende des Hülsenelements
- 12: proximales Ende des Hülsenelements
- 13: kreisförmiges Basiselement
- 14: radiale Außenfläche
- 14a: Vorsprung an der radialen Außenfläche
- 15: distales Ende des inneren Elements
- 16: proximales Ende des inneren Elements
- 17: Flanschelement
- 17a: Außenfläche des Flanschelements
- 17b: Auflagefläche des Flanschelements
- 18: Aufnahmeeinrichtung des Hülsenelements
- 19: erste Wandung der Aufnahmeeinrichtung
- 20: zweite Wandung der Aufnahmeeinrichtung
- 21: erster Bereich des Vorsprungs
- 21a: Außendurchmesser des ersten Bereichs
- 22: Innendurchmesser der Ausnehmung des inneren Elements
- 23: zweiter Bereich des Vorsprungs
- 23a: Außendurchmesser des weiten Bereichs
- 24: axiales Toleranzausgleichselement
- 25: kegelförmige Vorsprünge
- 26: umlaufender Ring
- 26a: Grundfläche des Rings
- 27: Rippen
- 28: weitere Ausnehmung des inneren Elements
- 29: erster Abschnitt der weiteren Ausnehmung
- 29a: Innendurchmesser des ersten Abschnitts der weiteren Ausnehmung
- 30: zweiter Abschnitt der weiteren Ausnehmung
- 30a: Innendurchmesser des zweiten Abschnitts der weiteren Ausnehmung
- M_{H}: Mittelachse des Hülsenelements
- M_{IE}: Mittelachse des inneren Elements
- M_{V}: Mittelachse des Verbindungselements
- R: radiale Richtung
- X: axiale Richtung
- X₁: distale Richtung
- X₂: proximale Richtung

## Patentansprüche

1. Stechmittelschutzvorrichtung (1) für eine Spritze (2) mit einem Spritzenkörper (3) und einem an dem distalen Ende (4) des Spritzenkörpers (3) angeordneten Stechmittel (5), umfassend ein sich entlang einer axialen Richtung (X) erstreckendes formstabiles Hülsenelement (6), welches zumindest teilweise ein sich entlang einer axialen Richtung (X) erstreckendes inneres Element (7) umschließt, wobei das innere Element (7) aus einem elastischen Material besteht und zumindest teilweise das Stechmittel (5) umschließen kann, **dadurch gekennzeichnet, dass**
ein mit dem Hülsenelement (6) verbundenes Verbindungselement (8) zumindest einen sich in axialer Richtung (X) erstreckenden Vorsprung (9) aufweist, welcher zumindest abschnittsweise in einer Ausnehmung (10) des inneren Elements (7) aufgenommen ist, so dass eine kraft- und / oder formschlüssige Verbindung zwischen dem Hülsenelement (6) und dem inneren Element (7) besteht.

2. Stechmittelschutzvorrichtung (1) nach Anspruch 1,
**dadurch gekennzeichnet, dass**
das Hülsenelement (6) im Wesentlichen als kreisförmiger Hohlzylinder ausgebildet ist und ein distales (11) sowie ein proximales (12) Ende aufweist, wobei das Verbindungselement (8) an dem distalen Ende (11) des Hülsenelements (6) angeordnet ist, wobei die Verbindung zwischen dem Hülsenelement (6) und dem Verbindungselement (8) eine form- und / oder stoffschlüssige Verbindung ist.

3. Stechmittelschutzvorrichtung (1) nach einem der Ansprüche 1 oder 2,
**dadurch gekennzeichnet, dass**
das innere Element (7) an seinem distalen Ende (15) ein sich in radialer Richtung (R) erstreckendes Flanschelement (17) aufweist, welches in einer Aufnahmeeinrichtung (18) des Hülsenelements (6) aufgenommen ist, wobei die Aufnahmeeinrichtung (18) an dem distalen Ende (11) des Hülsenelements (8) angeordnet ist und eine erste Wandung (19), welche sich ausgehend von dem distalen Ende (11) des Hülsenelements (8) in axialer Richtung (X) erstreckt und eine zweite Wandung (20), welche sich im Wesentlichen in radialer Richtung (R) erstreckt, aufweist.

4. Stechmittelschutzvorrichtung (1) nach einem der vorhergehenden Ansprüche, **dadurch gekennzeichnet, dass**
das Verbindungselement (8) ein kreisförmiges Basiselement (13) aufweist, an welchem der Vorsprung (9) angeordnet ist, wobei das kreisförmige Basiselement (13) eine radiale Außenfläche (14) aufweist, welche mit dem Hülsenelement (6) verbunden ist.

5. Stechmittelschutzvorrichtung (1) nach einem der vorhergehenden Ansprüche, **dadurch gekennzeichnet, dass**
der Vorsprung (9) an dem Verbindungselement (8) mittig bezüglich einer Mittelachse (M_{H}) des Hülsenelements (6) angeordnet ist und die Ausnehmung (10) mittig bezüglich einer Mittelachse (M_{IE}) des inneren Elements (7) angeordnet ist, wobei durch die jeweilige mittige Anordnung und die Verbindung zwischen dem Verbindungselement (8) und dem inneren Element (7) das innere Element (7) bezüglich des Hülsenelements (6) zentrierbar ist.

6. Stechmittelschutzvorrichtung (1) nach einem der Ansprüche 3 bis 5,
**dadurch gekennzeichnet, dass**
der Vorsprung (9) des Verbindungselements (8) konisch ausgebildet ist, wobei ein Außendurchmesser (21a) eines ersten Bereichs (21) des Vorsprungs (9) größer ist als ein Innendurchmesser (22) der Ausnehmung (10) des inneren Elements (7), wobei durch die Aufnahme des Vorsprungs (9) in der Ausnehmung (10) des inneren Elements (7) das innere Element (7) elastisch verformt ist, wobei durch diese Verformung das Flanschelement (17) des inneren Elements (7) in radialer Richtung (R) an die Aufnahmeeinrichtung (18) gedrückt ist.

7. Stechmittelschutzvorrichtung (1) nach einem der Ansprüche 4 bis 6,
**dadurch gekennzeichnet, dass**
an dem Basiselement (13) des Verbindungselements (8) zumindest ein axiales Toleranzausgleichselement (24) angeordnet ist, welches sich in axialer Richtung (X) zu dem inneren Element (7) hin erstreckt und das innere Element (7) kontaktiert.

8. Stechmittelschutzvorrichtung (1) nach Anspruch 7,
**dadurch gekennzeichnet, dass**
zumindest zwei axiale Toleranzausgleichselemente (24) an dem Basiselement (13) des Verbindungselements (8) angeordnet sind, welche als kegelförmige Vorsprünge (25) ausgestaltet sind, wobei die zumindest zwei axialen Toleranzausgleichselemente kreisförmig um den Vorsprung (9) angeordnet sind.

9. Stechmittelschutzvorrichtung (1) nach Anspruch 7,
**dadurch gekennzeichnet, dass**
das axiale Toleranzausgleichselement (24) als ein den Vorsprung (9) umlaufendes ringartiges Element (26) ausgestaltet ist, wobei das ringartige Element (26) sich in axialer Richtung (X) zu dem inneren Element (7) hin verjüngt.

10. Stechmittelschutzvorrichtung (1) nach einem der vorhergehenden Ansprüche, **dadurch gekennzeichnet, dass**
der Vorsprung (9) eine Außenfläche (9a) umfasst, an welcher in axialer Richtung verlaufende Rippen (27) angeordnet sind.

11. Vorfüllbare Spritze ausgestattet mit einer Stechmittelschutzvorrichtung (1) nach einem der vorhergehenden Ansprüche.

## Claims

1. Piercing tool protection device (1) for a syringe (2) having a syringe body (3) and a piercing tool (5) arranged at the distal end (4) of the syringe body (3), comprising a dimensionally stable sleeve element (6), which extends in an axial direction (X) and at least partially encloses an inner element (7) extending in an axial direction (X), the inner element (7) consisting of a resilient material and being able to at least partially enclose the piercing tool (5),
**characterised in that**
a connection element (8) connected to the sleeve element (6) has at least one protrusion (9) extending in the axial direction (X), which protrusion is received at least in portions in a recess (10) of the inner element (7) such that a force- and/or form-fitting connection exists between the sleeve element (6) and the inner element (7).

2. Piercing tool protection device (1) according to claim 1,
**characterised in that**
the sleeve element (6) is formed substantially as a circular hollow cylinder and has a distal (11) and a proximal (12) end, the connection element (8) being arranged at the distal end (11) of the sleeve element (6), the connection between the sleeve element (6) and the connection element (8) being a form-fitting and/or firmly bonded connection.

3. Piercing tool protection device (1) according to either claim 1 or claim 2,
**characterised in that**
the inner element (7) has at its distal end (15) a flange element (17) extending in the radial direction (R), which flange element is received in a receiving means (18) of the sleeve element (6), the receiving means (18) being arranged on the distal end (11) of the sleeve element (8) and having a first wall (19), which extends in the axial direction (X) starting from the distal end (11) of the sleeve element (8), and a second wall (20), which extends substantially in the radial direction (R).

4. Piercing tool protection device (1) according to any of the preceding claims, **characterised in that**
the connection element (8) has a circular base element (13) on which the protrusion (9) is arranged, the circular base element (13) having a radial outer surface (14) which is connected to the sleeve element (6).

5. Piercing tool protection device (1) according to any of the preceding claims, **characterised in that**
the protrusion (9) is arranged on the connection element (8) centrally with respect to a central axis (MH) of the sleeve element (6) and the recess (10) is arranged centrally with respect to a central axis (MIE) of the inner element (7), it being possible for the inner element (7) to be centred with respect to the sleeve element (6) owing to the respective central arrangement and the connection between the connection element (8) and the inner element (7).

6. Piercing tool protection device (1) according to any of claims 3 to 5,
**characterised in that**
the protrusion (9) of the connection element (8) is conical, an external diameter (21a) of a first region (21) of the protrusion (9) being greater than an internal diameter (22) of the recess (10) of the inner element (7), the inner element (7) being elastically deformed as a result of the protrusion (9) being received in the recess (10) of the inner element (7), this deformation meaning that the flange element (17) of the inner element (7) is pressed against the receiving means (18) in the radial direction (R).

7. Piercing tool protection device (1) according to any of claims 4 to 6,
**characterised in that**
at least one axial tolerance-compensation element (24) is arranged on the base element (13) of the connection element (8) and extends in the axial direction (X) towards the inner element (7) and contacts the inner element (7).

8. Piercing tool protection device (1) according to claim 7,
**characterised in that**
at least two axial tolerance-compensation elements (24) are arranged on the base element (13) of the connection element (8) and are designed as cone-shaped protrusions (25), the at least two axial tolerance-compensation elements being arranged in a circular manner around the protrusion (9).

9. Piercing tool protection device (1) according to claim 7,
**characterised in that**
the axial tolerance-compensation element (24) is designed as an annular element (26) that surrounds the protrusion (9), the annular element (26) tapering in the axial direction (X) towards the inner element (7).

10. Piercing tool protection device (1) according to any of the preceding claims,
**characterised in that**
the protrusion (9) comprises an outer surface (9a) on which ribs (27) extending in the axial direction are arranged.

11. Prefillable syringe equipped with a piercing tool protection device (1) according to any of the preceding claims.

## Revendications

1. Dispositif (1) de protection de moyen de perçage pour une seringue (2) comportant un corps de seringue (3) et un moyen de perçage (5) disposé à l'extrémité distale (4) du corps de seringue (3), comportant un élément manchon (6), dimensionnellement stable, qui s'étend le long d'une direction axiale (X) et entoure au moins partiellement un élément interne (7) s'étendant le long d'une direction axiale (X), l'élément interne (7) étant fait d'une matière élastique et pouvant au moins partiellement entourer le moyen de perçage (5),
**caractérisé par le fait que**
un élément de liaison (8) relié à l'élément manchon (6) présente au moins une saillie (9) s'étendant dans la direction axiale (X), saillie qui est reçue au moins par portions dans une cavité (10) de l'élément interne (7), de telle sorte qu'une liaison par friction et/ou par coopération de formes existe entre l'élément manchon (6) et l'élément interne (7).

2. Dispositif (1) de protection de moyen de perçage selon la revendication 1,
**caractérisé par le fait que**
l'élément manchon (6) est réalisé essentiellement en tant que cylindre creux circulaire et présente une extrémité distale (11) ainsi qu'une extrémité proximale (12), l'élément de liaison (8) étant disposé à l'extrémité distale (11) de l'élément manchon (6), la liaison entre l'élément manchon (6) et l'élément de liaison (8) étant une liaison par friction et/ou par coopération de formes.

3. Dispositif (1) de protection de moyen de perçage selon l'une des revendications 1 ou 2,
**caractérisé par le fait que**
l'élément interne (7) présente à son extrémité distale (15) un élément à bride (17) s'étendant dans la direction radiale (R), lequel est reçu dans un moyen de réception (18) de l'élément manchon (6), le moyen de réception (18) étant disposé sur l'extrémité distale (11) de l'élément manchon (6) et présentant une première paroi (19), qui s'étend dans la direction axiale (X) à partir de l'extrémité distale (11) de l'élément manchon (6), et une seconde paroi (20), qui s'étend sensiblement dans la direction radiale (R).

4. Dispositif (1) de protection de moyen de perçage selon l'une des revendications précédentes,
**caractérisé par le fait que**
l'élément de liaison (8) présente un élément de base circulaire (13), sur lequel la saillie (9) est disposée, l'élément de base circulaire (13) présentant une surface externe radiale (14), laquelle est reliée à l'élément manchon (6).

5. Dispositif (1) de protection de moyen de perçage selon l'une des revendications précédentes,
**caractérisé par le fait que**
la saillie (9) est disposée sur l'élément de liaison (8) de façon centrale par rapport à un axe central (MH) de l'élément manchon (6), et la cavité (10) est disposée de façon centrale par rapport à un axe central (MIE) de l'élément interne (7), l'élément interne (7) étant apte à être centré par rapport à l'élément manchon (6) du fait de l'agencement central respectif et de la liaison entre l'élément de liaison (8) et l'élément interne (7).

6. Dispositif (1) de protection de moyen de perçage selon l'une des revendications 3 à 5,
**caractérisé par le fait que**
la saillie (9) de l'élément de liaison (8) est réalisée conique, un diamètre externe (21a) d'une première zone (21) de la saillie (9) étant plus grand qu'un diamètre interne (22) de la cavité (10) de l'élément interne (7), l'élément interne (7) étant élastiquement déformé du fait de la réception de la saillie (9) dans la cavité (10) de l'élément interne (7), l'élément à bride (17) de l'élément interne (7) étant pressé sur le moyen de réception (18) dans la direction radiale (R) du fait de cette déformation.

7. Dispositif (1) de protection de moyen de perçage selon l'une des revendications 4 à 6,
**caractérisé par le fait que**
sur l'élément de base (13) de l'élément de liaison (8), au moins un élément de compensation de tolérance axial (24) est disposé, lequel s'étend dans la direction axiale (X) vers l'élément interne (7) et vient en contact avec l'élément interne (7).

8. Dispositif (1) de protection de moyen de perçage selon la revendication 7,
**caractérisé par le fait que**
au moins deux éléments de compensation de tolérance axiaux (24) sont disposés sur l'élément de base (13) de l'élément de liaison (8), lesquels sont configurés en tant que saillies (25) de forme conique, les au moins deux éléments de compensation de tolérance axiaux étant disposés de façon circulaire autour de la saillie (9).

9. Dispositif (1) de protection de moyen de perçage selon la revendication 7,
**caractérisé par le fait que**
l'élément de compensation de tolérance axial (24) est configuré en tant qu'élément annulaire (26) entourant la saillie (9), l'élément annulaire (26) s'effilant dans la direction axiale (X) vers l'élément interne (7).

10. Dispositif (1) de protection de moyen de perçage selon l'une des revendications précédentes,
**caractérisé par le fait que**
la saillie (9) comporte une surface externe (9a), sur laquelle sont disposées des nervures (27) s'étendant dans la direction axiale.

11. Seringue pré-remplissable équipée d'un dispositif (1) de protection de moyen de perçage selon l'une des revendications précédentes.
